**Europäisches Patentamt**

**European Patent Office**

**Office européen des brevets**

(19)

(11) Veröffentlichungsnummer: **0 217 182**
**B1**

(12) # EUROPÄISCHE PATENTSCHRIFT

(45) Veröffentlichungstag der Patentschrift:
**01.06.88**

(21) Anmeldenummer: **86112430.3**

(22) Anmeldetag: **08.09.86**

(51) Int. Cl.⁴: **C 07 C 53/12,** C 07 C 53/08,
C 07 C 51/42, C 07 C 51/573,
C 07 C 27/26

(54) **Verfahren zur Abtrennung von Jod und dessen Verbindungen aus den bei der Carbonylierung von Dimethylether, Methylacetat oder Methanol erhaltenen Carbonylierungsprodukten.**

(30) Priorität: **25.09.85 DE 3534070**

(43) Veröffentlichungstag der Anmeldung:
**08.04.87 Patentblatt 87/15**

(45) Bekanntmachung des Hinweises auf die Patenterteilung:
**01.06.88 Patentblatt 88/22**

(84) Benannte Vertragsstaaten:
**AT BE CH DE FR GB IT LI NL**

(56) Entgegenhaltungen:
**EP - A - 0 143 179**
**US - A - 4 102 922**

(73) Patentinhaber: **HOECHST AKTIENGESELLSCHAFT,**
**Postfach 80 03 20, D-6230 Frankfurt am Main 80 (DE)**

(72) Erfinder: **Erpenbach, Heinz, Dr., Oberbuschweg 22,**
**D-5000 Köln (DE)**
Erfinder: **Gehrmann, Klaus, Dr.,**
**Geschwister-Scholl-Strasse 32, D-5042 Erfstadt (DE)**
Erfinder: **Lork, Winfried,**
**Siegfried-von-Westerburg-Strasse 14, D-5042 Erfstadt**
**(DE)**
Erfinder: **Prinz, Peter, von-Geyr-Ring 104, D-5030 Hürth**
**(DE)**

## Beschreibung

Die Erfindung betrifft ein Verfahren zur Abtrennung von Jod und dessen Verbindungen aus den bei der Carbonylierung von Dimethylether, Methylacetat oder Methanol in Gegenwart jodhaltiger Katalysatoren erhaltenen Carbonylierungsprodukten Essigsäure, Acetanhydrid oder Ethylidendiacetat, wobei man zur Herabsetzung der Menge des die Carbonylierungsprodukte verunreinigenden Gesamtjods auf Gehalte unterhalb 20 ppb Jod, die Carbonylierungsprodukte bei Temperaturen von 50 bis 200° C mit Wasserstoff in Gegenwart mindestens eines Edelmetalls aus der Gruppe VIII des Periodensystems der Elemente als Katalysator behandelt und vom Katalysator abtrennt.

Ein derartiges Verfahren ist bereits aus der DE-A-33 31 548 bekannt. Bei diesem Verfahren zeigt sich, dass der Katalysator in seiner Aktivität ziemlich rasch nachlässt, so dass zur weiteren Erzielung einer befriedigenden Jodabtrennung die Behandlungstemperatur und/oder die Katalysatormenge erhöht werden müssen. Diese Massnahmen führen bei der $H_2$-Behandlung von mit Jod verunreinigtem Essigsäureanhydrid zur unerwünschten Bildung von Nebenprodukten, z. B. von Acetaldehyd, Essigsäure und Ethylidendiacetat. Die vorliegende Erfindung beschreibt nun ein Verfahren, das es ermöglicht, die genannten Nachteile des Verfahrens der DE-A-33 31 548 auszuschalten. Überraschenderweise gelingt es, schon durch einen geringen Zusatz von Kohlenmonoxid zum Reaktionssystem Wasserstoff/Edelmetall die Bildung der Nebenprodukte wie z. B. Acetaldehyd, Essigsäure und Ethylidendiacetat stark zurückzudrängen und eine wesentlich längere Wirksamkeit des Katalysators zu erzielen. Auch bei diesem Verfahren wird Jod in jeder Art von gebundener wie elementarer Form aus den zu reinigenden Carbonylierungsprodukten auf verfahrenstechnisch einfache Weise abgetrennt.

Im einzelnen ist das Verfahren der Erfindung gemäss den Patentansprüchen nunmehr dadurch gekennzeichnet, dass man die Carbonylierungsprodukte mit Gemischen aus Wasserstoff und Kohlenmonoxid behandelt. Je Mol Carbonylierungsprodukt setzt man bevorzugt je 0,001 bis 1 Mol Wasserstoff und Kohlenmonoxid ein.

Ebenso wie beim Verfahren der DE-A-33 31 548 kann man auch im Verfahren vorliegender Erfindung bevorzugt und wahlweise

a) Carbonylierungsprodukte mit weniger als 100 ppm Gesamtjod einsetzen,

b) das Edelmetall in feiner Verteilung auf einem Träger einsetzen, c) die Behandlung der Carbonylierungsprodukte während 0,2 bis 6 Stunden vornehmen,

d) die Behandlung der Carbonylierungsprodukte unter einem Druck von 0,5 bis 10 bar vornehmen,

e) 0,001 bis 1 Masse% Edelmetall, bezogen auf die eingesetzten Carbonylierungsprodukte, als Katalysator einsetzen.

Zur Fixierung der jodhaltigen Verunreinigungen setzt man zweckmässigerweise dem aus der letzten Reinigungsstufe des gesamten Carbonylierungsprozesses abgezogenen Produkt das Edelmetall zu, welches bevorzugt auf einem Katalysatorträger fein verteilt vorliegt. Am Edelmetall des Trägers fixierte jodhaltige Verbindungen werden vorzugsweise durch Abfiltrieren des Katalysators aus dem Reinprodukt abgetrennt. Je nach seinem Beladungsgrad wird das Edelmetall wieder zu erneutem Einsatz rückgeführt oder durch frischen Katalysator ersetzt.

Selbstverständlich kann der zur Eliminierung der jodhaltigen Verunreinigungen erforderliche Edelmetallkatalysator und das Wasserstoff/Kohlenmonoxidgemisch auch vor der letzten bzw. vorletzten Reinigungsstufe, in der der hochsiedende Rückstand bzw. der leichtsiedende Anteil vom Carbonylierungsprodukt entfernt wird, zugesetzt werden.

Nach dem Verfahren der Erfindung kann der Gesamtjodgehalt im behandelten Carbonylierungsprodukt bis unter die Nachweisbarkeitsgrenze von <5 ppb (=5 Gewichtsteile Jod pro 1 Milliarde ($10^9$) Gewichtsteile Carbonylierungsprodukt) gesenkt werden.

Die Herstellung des Katalysators ist nicht Gegenstand der Erfindung. Bevorzugt wird das Edelmetall in einer besonders feinverteilten und katalytisch wirksamen Form auf das Trägermaterial aufgebracht, indem der ausgewählte Träger zunächst mit einer Edelmetallsalzlösung getränkt und anschliessend mit einer ammoniakalischen Hydrazinlösung behandelt wird. Dabei fällt das Edelmetall in sehr feinverteilter Form auf dem Träger aus und ist nach dem Waschen bis zum Neutralpunkt und anschliessendem Trocknen einsatzbereit. Auch die Freisetzung des Edelmetalls durch Reduktion mit Wasserstoff bei erhöhter Temperatur ist möglich.

Als Träger für das Edelmetall können u.a. Kieselsäure oder Aluminiumoxid dienen. Die Korngrösse des Trägers bewegt sich je nach Einsatzart, z. B. als Fest- oder Wirbelbettkatalysator, in einem weiten Bereich und liegt dementsprechend zwischen 0,01 und 10 mm. Desgleichen umfasst seine innere Oberfläche (bestimmt nach BET) den Bereich zwischen 1 und 700 m²/g. Die Variationsbreite der Konzentration des auf dem Träger aufgetragenen Edelmetalls ist ebenfalls gross, bevorzugt wird sie jedoch zwischen 0,5 und 5 Masse% gehalten. Die Abtrennung des Carbonylierungsproduktes vom Edelmetallkatalysator erfolgt vorzugsweise durch Filtration.

Der verwendete Wasserstoff und das Kohlenmonoxid werden bevorzugt in reiner Form eingesetzt. Beide Gase können aber auch durch z. B. Stickstoff, Argon und andere inerte Komponenten verunreinigt sein.

Als Edelmetalle eignen sich Ruthenium, Osmium, Iridium, besonders jedoch Rhodium, Palladium und Platin. Das Verfahren der Erfindung kann sowohl in diskontinuierlicher als auch kontinuierlicher Betriebsweise durchgeführt werden.

Die analytische Bestimmung des Gesamtjods erfolgt durch die jodkatalysierte Reaktion zwischen Arsen- und Cerionen mit photometrischer Endbestimmung des Cers.

*Beispiel 1:*

200 g eines Carbonylierungsproduktes aus Essigsäure (19,1 Masse%), Essigsäureanhydrid (80,7 Masse%) und Ethylidendiacetat (0,2 Masse%), verunreinigt mit 2 ppm Gesamtjod, werden mit 0,15 g Palladium, welches auf 10 g $SiO_2$ (Korngrösse 0,01-0,2 mm, BET-Oberfläche 350 m²/g) feinverteilt aufgezogen ist, versetzt und mit 8 l/h eines Gemisches aus 50 Vol.-% Wasserstoff und 50 Vol.-% Kohlenmonoxid bei einer Temperatur von 120° C begast. Nach einer Begasungszeit von 5 Stunden wird die Zufuhr des Wasserstoff/Kohlenmonoxid-Gemisches abgestellt, das Carbonylierungsprodukt filtriert und analysiert. Es wird ein Gesamtjodwert von <5 ppb gefunden, woraus sich eine Jodabtrennung von >99,8% errechnet. Die gaschromatografische (GC)-Analyse zeigt mit 19,15 Masse% Essigsäure, 80,6 Masse% Essigsäureanhydrid und 0,25 Masse% Ethylidendiacetat eine unwesentliche Verschiebung der ursprünglichen Zusammensetzung des behandelten Carbonylierungsproduktes an. Der Ausbeuteverlust an Essigsäureanhydrid beträgt nur 0,1%.

*Beispiel 2:*

Der in Beispiel 1 verwendete Pd-Katalysator wird über eine Betriebszeit von 200 Stunden mit insgesamt 8 kg Carbonylierungsprodukt der Zusammensetzung 19,1 Masse% Essigsäure, 80,7 Masse% Essigsäureanhydrid und 0,2 Masse% Ethylidendiacetat, welches mit 2 ppm Gesamtjod verunreinigt ist, sowie 8 l/h eines Gasgemisches aus 50 Vol.-% Wasserstoff und 50 Vol.-% Kohlenmonoxid bei 120° C in Berührung gebracht. Das behandelte und filtrierte Carbonylierungsprodukt wird sowohl nach einer Laufzeit von 100 als auch 200 Stunden analysiert. Dabei ergeben sich jeweils Gesamtjodwerte von <5 ppb, was einer Jodentfernung von >99,8% entspricht. Die durch GC-Angaben ermittelte Zusammensetzung zeigt mit 19,15 Masse% Essigsäure, 80,6 Masse% Essigsäureanhydrid und 0,25 Masse% Ethylidendiacetat über die gesamte Betriebszeit konstante Werte an.

*Beispiel 3 (Vergleichsbeispiel)*

200 g eines Carbonylierungsgemisches aus Essigsäure (19,1 Masse%), Essigsäureanhydrid (80,7 Masse%) und Ethylidendiacetat (0,2 Masse%) verunreinigt mit 2 ppm Gesamtjod, wird mit 0,15 g Palladium, welches auf 10 g $SiO_2$ (Korngrösse 0,01-0,2 mm, BET-Oberfläche 350 m²/g) fein verteilt aufgezogen ist, versetzt und mit 4 l/h Wasserstoff bei einer Temperatur von 120° C begast. Nach einer Begasungszeit von 5 Stunden wird die Wasserstoffzufuhr abgestellt, das Carbonylierungsprodukt filtriert und analysiert. Es wird ein Gesamtjodwert von <5 ppb gefunden, woraus sich eine Jodabtrennung von >99,8% errechnet. Die GC-Analyse zeigt jedoch mit 20,0 Masse% Essigsäure, 77,7 Masse% Essigsäureanhydrid und 2,3 Masse% Ethylidendiacetat eine Reduzierung des Essigsäureanhydrid- und eine Zunahme des Essigsäure- und Ethylidendiacetat-Gehaltes und damit eine Verschiebung der ursprünglichen Zusammensetzung. Der Ausbeuteverlust an Essigsäureanhydrid beträgt nach dieser Behandlungsdauer 3,0%.

*Beispiel 4 (Vergleichsbeispiel)*

Der in Beispiel 3 eingesetzte Pd-Katalysator wird über eine Laufzeit von 200 Stunden mit insgesamt 8 kg Carbonylierungsprodukt der Zusammensetzung 19,1 Masse% Essigsäure, 80,7 Masse% Essigsäureanhydrid und 0,2 Masse% Ethylidendiacetat, welches mit 2 ppm Gesamtjod verunreinigt ist, sowie 4 l/h Wasserstoff bei einer Temperatur von 120° C in Berührung gebracht. Das behandelte und filtrierte Carbonylierungsprodukt wird nach einer Betriebszeit von 100 und 200 Stunden analysiert. Dabei wird bereits nach 100 Stunden Laufzeit der Jodgehalt nur noch bis auf 0,4 ppm abgebaut, entsprechend einer Entfernungsrate von 80%. Zur Erzielung einer besseren Jodentfernung wird daher nach 100 Stunden Laufzeit die Temperatur im Reaktor auf 130° C angehoben. Dieses bewirkt eine Reduzierung des Jodgehaltes im Reaktionsprodukt bis auf einen Wert von 70 ppb, entsprechend einer Jodentfernung von 96,5%. Nach weiteren 100 Betriebsstunden (entsprechend einer Gesamtversuchszeit von 200 Stunden) wird der Versuch beendet und das filtrierte Produkt analysiert. Während der Jodgehalt nach dieser Zeit nur noch von 2 ppm auf 0,3 ppm reduziert werden kann, entsprechend einer Jodentfernung von 85%, zeigt die GC-Analyse eine deutliche Veränderung des eingesetzten Carbonylierungsproduktes. Die Zusammensetzung beträgt 20,5 Masse% Essigsäure, 76,0 Masse% Essigsäureanhydrid, 3,5 Masse% Ethylidendiacetat und 600 ppm Acetaldehyd. Der Verlust an Anhydrid nach dieser Behandlungsmethode beträgt 4,7%, bezogen auf den Einsatz.

*Beispiel 5:*

200 g Essigsäureanhydrid, verunreinigt mit 0,6 ppm Gesamtjod werden mit 0,15 g Palladium, welches auf 10 g Kieselgel (Korngrösse 0,01-0,2 mm, BET-Oberfläche 580 m²/g) fein verteilt aufgezogen ist, versetzt und mit 10 l/h eines Gemisches aus 95 Vol.-% Wasserstoff und 5 Vol.-% Kohlenmonoxid bei einer Temperatur von 100° C begast. Nach einer Begasungszeit von 3 Stunden wird das Essigsäureanhydrid vom Katalysator filtriert und analysiert. Aus dem gefundenen Jodwert von <5 ppb errechnet sich eine Jodabtrennung von >99,2%. Durch GC-Analyse konnte im Essigsäureanhydrid keine Nebenproduktbildung, wie z. B. Ethylidendiacetat, festgestellt werden.

**Patentansprüche**

1. Verfahren zur Abtrennung von Jod und dessen Verbindungen aus den bei der Carbonylierung von Dimethylether, Methylacetat oder Methanol in Gegenwart jodhaltiger Katalysatoren erhaltenen Carbonylierungsprodukten Essigsäure, Acetanhydrid oder Ethylidendiacetat, wobei man zur Herabsetzung der Menge des die Carbonylierungsprodukte verunreinigenden Gesamtjods auf Gehalte unterhalb 20 ppb Jod, die Carbonylierungsprodukte bei Temperaturen von 50 bis 200° C mit Wasserstoff in Gegenwart mindestens eines Edelmetalls aus der Gruppe VIII des Periodensystems der Elemente als Katalysator behandelt und vom Katalysator abtrennt, *dadurch gekennzeichnet,* dass man bei der Behandlung der Carbonylierungsprodukte anstelle von Wasserstoff nunmehr Gemische aus Wasserstoff und Kohlenmonoxid einsetzt.

2. Verfahren nach Anspruch 1, *dadurch gekennzeichnet,* dass man je Mol Carbonylierungsprodukt je 0,001 bis 1 Mol Wasserstoff und Kohlenmonoxid einsetzt.

## Claims

1. Process for separating iodine and its compounds from the carbonylation products acetic acid, acetic anhydride or ethylidene diacetate obtained on subjecting dimethylether, methyl acetate or methanol to a carbonylation reaction in the presence of catalysts containing iodine, wherein in order to reduce the total iodine contaminating the carbonylation products to a content of less than 20 ppb iodine, the carbonylation products are treated at temperatures of 50-200° C with hydrogen in the presence of at least one noble metal belonging to group VIII of the Periodic System of the elements as a catalyst, and separated from the catalyst, which comprises substituting in the treatment of the carbonylation products a hydrogen/carbon monoxide-mixture for hydrogen.

2. Process as claimed in claim 1, wherein 0.001-1 mol each of hydrogen and carbon monoxide is used per mol of carbonylation product.

## Revendications

1. Procédé de séparation de l'iode et de ses composés des produits de carbonylation, acide acétique, anhydride acétique ou diacétate d'éthylidène, obtenus dans la carbonylation de l'éther diméthylique, de l'acétate de méthyle ou du méthanol en présence de catalyseurs contenant de l'iode, dans lequel, pour réduire la quantité de l'iode total contaminant les produits de carbonylation à une teneur inférieure à 20 ppb, on traite les produits de carbonylation à des températures de 50-200° C par l'hydrogène en présence d'au moins un métal noble du groupe VIII de la Classification Périodique des Eléments comme catalyseur et on les sépare du catalyseur, caractérisé en ce que, dans le traitement des produits de carbonylation, on utilise au lieu d'hydrogène des mélanges d'hydrogène et de monoxyde de carbone.

2. Procédé selon la revendication 1, caractérisé en ce que l'on utilise chaque fois 0,001-1 mol d'hydrogène et de monoxyde de carbone par mole de produit de carbonylation.